(19) 

Europäisches Patentamt

European Patent Office

Office européen des brevets

(11) **EP 4 174 089 A1**

(12) **EUROPEAN PATENT APPLICATION**
published in accordance with Art. 153(4) EPC

(43) Date of publication:
**03.05.2023  Bulletin 2023/18**

(21) Application number: **21849058.9**

(22) Date of filing: **30.07.2021**

(51) International Patent Classification (IPC):
*C07K 16/28* (2006.01)     *C12N 15/13* (2006.01)
*C07K 19/00* (2006.01)     *C12N 15/62* (2006.01)
*C12N 15/867* (2006.01)     *C12N 5/10* (2006.01)
*A61K 39/00* (2006.01)     *A61P 35/00* (2006.01)

(86) International application number:
**PCT/CN2021/109849**

(87) International publication number:
**WO 2022/022716 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.07.2020  CN 202010760969**

(71) Applicant: **Beijing Neurosurgical Institute
Beijing 100070 (CN)**

(72) Inventors:
• **ZHANG, Wei
Beijing 100070 (CN)**

• **JIANG, Tao
Beijing 100070 (CN)**
• **ZHAI, You
Beijing 100070 (CN)**
• **LI, Guanzhang
Beijing 100070 (CN)**

(74) Representative: **Prüfer & Partner mbB
Patentanwälte · Rechtsanwälte
Sohnckestraße 12
81479 München (DE)**

Remarks:
The complete document including Reference Table(s) and the Sequence Listing(s) can be downloaded from the EPO website

(54) **ANTI-TIM3 SINGLE-CHAIN ANTIBODY AND USE THEREOF IN PREPARING MEDICINE FOR TREATING TUMORS**

(57)     Disclosed is an anti-TIM3 single-chain antibody. The amino acid sequence of the anti-TIM3 single-chain antibody comprises a sequence shown in SEQ ID NO. 1. T lymphocytes expressing the anti-TIM3 single-chain antibody can effectively kill tumor cells.

Fig. 1

EP 4 174 089 A1

**Description**

**TECHNICAL FIELD**

The disclosure relates to the field of medical biotechnology, in particular to a chimeric antigen receptor targeting TIM3, an encoding nucleic acid, an expression vector, a cell, a pharmaceutical composition and use thereof.

**BACKGROUND**

Chimeric antigen receptor (CAR) is a synthetic T cell receptor consisting of an antigen binding domain, a transmembrane domain and an intracellular signaling domain. The antigen binding domain is located outside a T cell membrane, includes a single-chain antibody or ligand, and is used for specifically binding to a target antigen. The intracellular signaling domain is located within the T cell membrane, and is used for transmitting signals into a T cell to stimulate the immune response of the T cell.

The CAR can target and identify the target antigen on the surface of a tumor cell, so the T cell expressing the CAR can be used for targeting and killing a tumor cell. However, the existing T cell expressing the CAR is still weak in killing the tumor cell.

**SUMMARY**

The objective of the disclosure is to overcome the problem that the existing T cell expressing a CAR is still weak in killing a tumor cell, and to provide an anti-TIM3 single-chain antibody.

In order to achieve the above objective, in a first aspect, the disclosure provides an anti-TIM3 single-chain antibody, the amino acid sequence of which includes a sequence shown in SEQ ID NO. 1.

In a second aspect, the disclosure provides a nucleic acid encoding the anti-TIM3 single-chain antibody according to the first aspect; and preferably, the nucleic acid has a nucleotide sequence shown in SEQ ID NO. 2.

In a third aspect, the disclosure provides a fusion protein containing an antigen binding domain, a transmembrane domain and an intracellular signaling domain that are sequentially linked; the amino acid sequence of the intracellular signaling domain includes the amino acid sequence of the anti-TIM3 single-chain antibody according to the first aspect.

Preferably, the antigen binding domain includes an anti-CD44 single-chain antibody and/or an anti-CD133 single-chain antibody, and the intracellular signaling domain includes the anti-TIM3 single-chain antibody.

More preferably, the fusion protein has an amino acid sequence shown in SEQ ID NO. 3.

In a fourth aspect, the disclosure provides a fusion nucleic acid encoding the fusion protein according to the third aspect.

Preferably, the fusion nucleic acid has a nucleotide sequence shown in SEQ ID NO. 4.

In a fifth aspect, the disclosure provides an expression vector, the expression vector is inserted with an expression cassette, the expression cassette includes a first nucleic acid fragment encoding an antigen binding molecule and a second nucleic acid fragment encoding an intracellular signaling molecule, the intracellular signaling molecule contains the anti-TIM3 single-chain antibody according to the first aspect, and an IRES element or a 2A peptide coding sequence is inserted between the first nucleic acid fragment and the second nucleic acid fragment.

Preferably, the expression cassette is the fusion nucleic acid according to the fourth aspect.

In a sixth aspect, the disclosure provides a cell expressing a chimeric antigen receptor, the cell expressing a chimeric antigen receptor is obtained by transfection of the expression vector according to the fifth aspect by a host cell, and the chimeric antigen receptor contains the anti-TIM3 single-chain antibody according to the first aspect.

Optionally, the host cell is a T cell.

In a seventh aspect, the disclosure provides use of the anti-TIM3 single-chain antibody according to the first aspect, the nucleic acid according to the second aspect, the fusion protein according to the third aspect, the fusion nucleic acid according to the fourth aspect, the expression vector according to the fifth aspect, and the cell expressing a chimeric antigen receptor according to the sixth aspect in preparing a medicine for treating a tumor.

Optionally, the tumor is glioma.

In an eighth aspect, the disclosure provides a pharmaceutical composition, an active ingredient of which includes the cell expressing an anti-TIM3 chimeric antigen receptor according to the sixth aspect.

According to the above technical solutions, the anti-TIM3 single-chain antibody provided by the disclosure can effectively improve the ability of T lymphocytes to kill tumor cells, so the T lymphocytes expressing the anti-TIM3 single-chain antibody can effectively kill tumor cells.

Other features and advantages of the disclosure will be described in detail in the following specific embodiments.

## BRIEF DESCRIPTION OF THE DRAWINGS

The accompanying drawings are intended to provide a further understanding of the disclosure, constitute a part of the description, and are used for interpreting the disclosure together with the following specific embodiments, rather than limiting the disclosure. In the figures:

Fig. 1 is a flow cytometer test result diagram of CAR-T 1 cells according to an embodiment of the disclosure;
Fig. 2 is a flow cytometer test result diagram of CAR-T 2 cells according to an embodiment of the disclosure; and
Fig. 3 is a flow cytometer test result diagram of CAR-T 3 cells according to an embodiment of the disclosure.

## DETAILED DESCRIPTION

The specific embodiments of the disclosure will be described in detail below with reference to the accompanying drawings. It should be understood that the specific embodiments described herein are merely used for illustrating and interpreting the disclosure, rather than limiting the disclosure.

A first aspect of the disclosure provides an anti-TIM3 single-chain antibody, the amino acid sequence of the anti-TIM3 single-chain antibody includes a sequence shown in SEQ ID NO. 1.

Where the sequence shown in SEQ ID NO. 1 is as follows:

QVQLQQSGPGLVKPSHTLSLTCTVSGGSISSGGYYWSWTRQHPGMGLEWIGYI
SYSGSIYYTPSLKSRLTISVDTSKNQFSLKLSSVTAADTAVYYCASLDSWGSNRDYWGQ
GTLVTVSSGGGGSEIVLTQSPATLSLSPGERATLSCRASQSVSSYLAWYQQKPGQAPRLLI
YDASNRATGIPARFSGSGSGTDFTLTISSLEPEDFAVYYCQQRSNWPLTFGGGTKVEIK.

T cell immunoglobulin mucin-3 (TIM3) is an important tumor immunoassay site, is widely expressed on tumor infiltrating T lymphocytes, and can inhibit the tumor immunocompetence of tumor infiltrating T lymphocytes. The anti-TIM3 single-chain antibody designed for TIM3 can effectively block TIM3 pathways in T lymphocytes, enhance the tumor immunocompetence of T lymphocytes, and enhance the ability of T lymphocytes to kill tumor cells.

The inventors of the disclosure found that T lymphocytes expressing the above-mentioned anti-TIM3 single-chain antibody can effectively kill tumor cells.

A second aspect of the disclosure provides a nucleic acid encoding the anti-TIM3 single-chain antibody according to the first aspect. Preferably, the nucleic acid has a nucleotide sequence shown in SEQ ID NO. 2.

Where the nucleotide sequence shown in SEQ ID NO. 2 is as follows:

caggtgcagctacagcagtcgggcccaggactggtgaagccttcacacaccctgtccctcacctgcactgtctctggtggct
ccatcagcagtggtggttactactggagttggacccgtcagcacccagggatgggcctggagtggattggatacatctcttacagtggga
gtatctattacactccgtccctcaagagtcgacttaccatatcagtggacacgtctaagaaccagttctccctgaagctgagctctgtgactgc
cgcggacacggccgtatattactgtgcgagtttggattcctggggatctaaccgtgactactggggccagggaaccctggtcaccgtctcg
agtggaggtggtggatccgaaattgtgttgacgcagtctccagccaccctgtctttgtctccaggggaaagagccaccctctcctgcaggg
ccagtcagagtgttagcagctacttagcctggtaccaacagaaacctggccaggctcccaggctcctcatctatgatgcatccaacagggc
cactggcatcccagccaggttcagtggcagtgggtctgggacagacttcactctcaccatcagcagcctagagcctgaagattttgcagttt
attactgtcagcagcgtagcaactggccgctcactttcggcggagggaccaaggtggagattaag.

A third aspect of the disclosure provides a fusion protein containing an antigen binding domain, a transmembrane domain and an intracellular signaling domain that are sequentially linked. The amino acid sequence of the intracellular signaling domain includes the amino acid sequence of the anti-TIM3 single-chain antibody according to the first aspect. Preferably, the antigen binding domain includes an anti-CD44 single-chain antibody and/or an anti-CD133 single-chain antibody, and the intracellular signaling domain includes the anti-TIM3 single-chain antibody. More preferably, the fusion protein has an amino acid sequence shown in SEQ ID NO. 3.

Where the fusion protein shown in SEQ ID NO. 3 consists of an anti-CD44 single-chain antibody, an anti-CD133 single-chain antibody, a CD28, a CD3, a T2A, and an anti-TIM3 single-chain antibody.

Where the amino acid sequence shown in SEQ ID NO. 3 is as follows:

QVQLVESGGGVVQPGRSLRLSCAASGFTFSSYGMHWVRQAPGKGLEWVAVI WYDGSNKFYADSVKGRFTISRDNSKNTLYLQMNSLRAEDTAVYYCARRSDYRGYYGM DVWGQGTTVTVSSGSTSGGGSGGGSGGGGSSEIVLTQSPATLSLSPGERATLSCRASQSV INYLAWYQQKPGQAPRLLIYDASNRASGIPARFSGSGSGTDFTLTISSLEPEDFAVYYCQQ RRNWPLTFGGGTKVEIKGGGGSGGGGSGGGGSQVQLVQSGAEVKKPGASVKVSCKAS GYTFTDFEMHWVRQAPGQGLEWMGDIDPGTGDTAYNLKFKGRVTMTTDTSTSTAYME LRSLRSDDTAVYYCALGAFVYWGQGTLVTVSSGSTSGGGSGGGSGGGGSSDVVMTQS PLSLPVTPGEPASISCRSSQSLANSYGNTYLSWYLQKPGQSPQLLIYGISNRFSGVPDRFS GSGSGTDFTLKISRVEAEDVGVYYCLQGTHQPYTFGQGTKLEIKTTTPAPRPPTPAPTIAS QPLSLRPEACRPAAGGAVHTRGLDFACDIYIWAPLAGTCGVLLLSLVITLYCRSKRSRLL HSDYMNMTPRRPGPTRKHYQPYAPPRDFAAYRSRVKFSRSADAPAYKQGQNQLYNELN LGRREEYDVLDKRRGRDPEMGGKPRRKNPQEGLYNELQKDKMAEAYSEIGMKGERRR

GKGHDGLYQGLSTATKDTYDALHMQALPPREGRGSLLTCGDVEENPGPMTNKCLLQIA LLLCFSTTALSQVQLQQSGPGLVKPSHTLSLTCTVSGGSISSGGYYWSWTRQHPGMGLE WIGYISYSGSIYYTPSLKSRLTISVDTSKNQFSLKLSSVTAADTAVYYCASLDSWGSNRD YWGQGTLVTVSSGGGGSEIVLTQSPATLSLSPGERATLSCRASQSVSSYLAWYQQKPGQ APRLLIYDASNRATGIPARFSGSGSGTDFTLTISSLEPEDFAVYYCQQRSNWPLTFGGGTK VEIK.

A fourth aspect of the disclosure provides a fusion nucleic acid encoding the fusion protein according to the third aspect. Preferably, the fusion nucleic acid has a nucleotide sequence shown in SEQ ID NO. 4.

Where the nucleotide sequence shown in SEQ ID NO. 4 is used for encoding the amino acid sequence shown in SEQ ID NO. 3.

Where the nucleotide sequence shown in SEQ ID NO. 4 is as follows:

caggtgcagctggtggagtctggggggaggcgtggtccagcctggggaggtccctgagactctcctgtgcagcgtctggattc

accttcagtagctatggcatgcactgggtccgccaggctccaggcaaggggctggagtgggtggcagttatatggtatgatggaagtaat

aaattctatgcagactccgtgaagggccgattcaccatctccagagacaattccaagaacacgctgtatctgcaaatgaacagcctgagag

ccgaggacacggctgtgtattactgtgcgaggagaagtgactacaggggctactacggtatggacgtctggggccaagggaccacggt

caccgtctcctcaggcagtactagcggtggtggctccggggggcggttccggtggggggcggcagcagcgaaattgtgttgacacagtctc

cagccaccctgtctttgtctccaggggaaagagccaccctctcctgcagggccagtcagagtgttatcaactacttagcctggtaccaaca

gaaacctggccaggctcccaggctcctcatctatgatgcatccaacagggcctctggcatcccagccaggttcagtggcagtgggtctgg

gacagacttcactctcaccatcagcagcctagagcctgaagattttgcagtttattactgtcagcagcgtcgcaactggccgctcactttcgg

cggagggaccaaggtggagatcaaaggaggtggtggatccggaggtggtggctccggaggtggtggatcccaggttcagctggtgca

gtctggagctgaggtgaagaagcctggggcctcagtgaaggtctcctgcaaggcttctggttacacctttaccgactttgaaatgcactgg

gtgcgacaggcccctggacaagggcttgagtggatgggagatattgatcctggaactggtgatactgcctacaatctgaagttcaagggc

agagtcaccatgaccacagacacatccacgagcacagcctacatggagctgaggagcctgaggtctgacgacacggccgtgtattactg

tgcgttggggggcctttgtttactggggccagggaaccctggtcaccgtctcctcaggcagtactagcggtggtggctccggggggcggttc

cggtggggggcggcagcagcgatgttgtgatgactcagtctccactctccctgcccgtcacccctggagagccggcctccatctcctgcag

gtctagtcagagtcttgcaaacagttatgggaacacctatttgtcttggtacctgcagaagccagggcagtctccacagctcctgatctatgg

gatttccaacagattttctggggtccctgacaggttcagtggcagtggatcaggcacagatttttacactgaaaatcagcagagtggaggctg

aggacgttggggtttattactgcttacaaggtacacatcagccgtacacgtttggccagggggaccaagctggagatcaaaaccactacccc

agcaccgaggccacccaccccggctcctaccatcgcctcccagcctctgtccctgcgtccggaggcatgtagacccgcagctggtggg

gccgtgcatacccggggtcttgacttcgcctgcgatatctacatttgggcccctctggctggtacttgcggggtcctgctgctttcactcgtga

tcactctttactgtaggagtaagaggagcaggctcctgcacagtgactacatgaacatgactccccgccgccccgggcccacccgcaag

cattaccagccctatgccccaccacgcgacttcgcagcctatcgctcccgcgtgaaattcagccgcagcgcagatgctccagcctacaag

caggggcagaaccagctctacaacgaactcaatcttggtcggagagaggagtacgacgtgctggacaagcggagaggacgggaccc

agaaatgggcgggaagccgcgcagaaagaatccccaagagggcctgtacaacgagctccaaaaggataagatggcagaagcctata

gcgagattggtatgaaaggggaacgcagaagaggcaaaggccacgacggactgtaccagggactcagcaccgccaccaaggacacc

tatgacgctcttcacatgcaggccctgccgcctcgggagggaagggggttctctcctgacctgcggcgatgtggaggagaacccccggacc

catgaccaacaagtgtctcctccaaattgctctcctgttgtgcttctccactacagctctttcccaggtgcagctacagcagtcgggcccagg

actggtgaagccttcacacaccctgtccctcacctgcactgtctctggtggctccatcagcagtggtggttactactggagttggacccgtca

gcacccagggatgggcctggagtggattggatacatctcttacagtggggagtatctattacactccgtccctcaagagtcgacttaccatatc

agtggacacgtctaagaaccagttctccctgaagctgagctctgtgactgccgcggacacggccgtatattactgtgcgagtttggattcct

ggggatctaaccgtgactactggggccagggaaccctggtcaccgtctcgagtggaggtggtggatccgaaattgtgttgacgcagtctc

cagccaccctgtctttgtctccaggggaaagagccaccctctcctgcagggccagtcagagtgttagcagctacttagcctggtaccaaca

gaaacctggccaggctcccaggctcctcatctatgatgcatccaacagggccactggcatcccagccaggttcagtggcagtgggtctgg

gacagacttcactctcaccatcagcagcctagagcctgaagattttgcagtttattactgtcagcagcgtagcaactggccgctcactttcgg

cggagggaccaaggtggagattaag.

A fifth aspect of the disclosure provides an expression vector, the expression vector is inserted with an expression cassette, the expression cassette includes a first nucleic acid fragment encoding an antigen binding molecule and a second nucleic acid fragment encoding an intracellular signaling molecule, the intracellular signaling molecule contains the anti-TIM3 single-chain antibody according to the first aspect, and an IRES element or a 2A peptide coding sequence is inserted between the first nucleic acid fragment and the second nucleic acid fragment; and preferably, the expression cassette is the fusion nucleic acid according to the fourth aspect.

A sixth aspect of the disclosure provides a cell expressing a chimeric antigen receptor, the cell expressing a chimeric antigen receptor is obtained by transfection of the expression vector according to the fifth aspect by a host cell, and the chimeric antigen receptor contains the anti-TIM3 single-chain antibody according to the first aspect. Optionally, the host cell is a T cell.

A seventh aspect of the disclosure provides use of the anti-TIM3 single-chain antibody according to the first aspect, the nucleic acid according to the second aspect, the fusion protein according to the third aspect, the fusion nucleic acid according to the fourth aspect, the expression vector according to the fifth aspect, and the cell expressing a chimeric antigen receptor according to the sixth aspect in preparing a medicine for treating a tumor. Optionally, the tumor is glioma.

An eighth aspect of the disclosure provides a pharmaceutical composition, an active ingredient of which includes the cell expressing an anti-TIM3 chimeric antigen receptor according to the sixth aspect.

The disclosure is further described by the following examples, but the disclosure is not limited thereby.

Example 1

This example was used to explain the construction of expression vectors.

(1) Nucleotide sequences shown in SEQ ID NO. 4 and SEQ ID NO. 5 were respectively synthesized by using a full sequence synthesis method. The nucleotide sequence shown in SEQ ID NO. 4 was used for encoding a fusion protein shown in SEQ ID NO. 3; and the nucleotide sequence shown in SEQ ID NO. 5 was used for encoding a fusion protein shown in SEQ ID NO. 6. The composition of the fusion proteins shown in SEQ ID NO. 3 and SEQ ID NO. 6 was as follows:

G1: the fusion protein shown in SEQ ID NO. 3 consisted of an anti-CD44 single-chain antibody, an anti-CD133 single-chain antibody, a CD28, a CD3, a T2A, and an anti-TIM3 single-chain antibody;
G2: the fusion protein shown in SEQ ID NO. 6 consisted of an anti-CD44 single-chain antibody, an anti-CD133 single-chain antibody, a CD28, and a CD3.

Where the nucleotide sequence shown in SEQ ID NO. 5 was as follows:

caggtgcagctggtggagtctggggggaggcgtggtccagcctggggaggtccctgagactctcctgtgcagcgtctggattc

accttcagtagctatggcatgcactgggtccgccaggctccaggcaaggggctggagtgggtggcagttatatggtatgatggaagtaat

aaattctatgcagactccgtgaagggccgattcaccatctccagagacaattccaagaacacgctgtatctgcaaatgaacagcctgagag

ccgaggacacggctgtgtattactgtgcgaggagaagtgactacaggggctactacggtatggacgtctggggccaagggaccacggt

caccgtctcctcaggcagtactagcggtggtggctccggggggcggttccggtgggggcggcagcagcgaaattgtgttgacacagtctc

cagccaccctgtctttgtctccaggggaaagagccaccctctcctgcagggccagtcagagtgttatcaactacttagcctggtaccaaca

gaaacctggccaggctcccaggctcctcatctatgatgcatccaacagggcctctggcatcccagccaggttcagtggcagtgggtctgg

gacagacttcactctcaccatcagcagcctagagcctgaagattttgcagtttattactgtcagcagcgtcgcaactggccgctcactttcgg

cggagggaccaaggtggagatcaaaggaggtggtggatccggaggtggtggctccggaggtggtggatcccaggttcagctggtgca

gtctggagctgaggtgaagaagcctggggcctcagtgaaggtctcctgcaaggcttctggttacacctttaccgactttgaaatgcactgg

gtgcgacaggcccctggacaagggcttgagtggatgggagatattgatcctggaactggtgatactgcctacaatctgaagttcaagggc

agagtcaccatgaccacagacacatccacgagcacagcctacatggagctgaggagcctgaggtctgacgacacggccgtgtattactg

tgcgttggggggcctttgtttactggggccagggaacccctggtcaccgtctcctcaggcagtactagcggtggtggctccggggggcggttc

cggtggggggcggcagcagcgatgttgtgatgactcagtctccactctccctgcccgtcacccctggagagccggcctccatctcctgcag

gtctagtcagagtcttgcaaacagttatgggaacacctatttgtcttggtacctgcagaagccagggcagtctccacagctcctgatctatgg

gatttccaacagattttctggggtccctgacaggttcagtggcagtggatcaggcacagattttacactgaaaatcagcagagtggaggctg

aggacgttggggtttattactgcttacaaggtacacatcagccgtacacgtttggccagggggaccaagctggagatcaaaaccactacccc

agcaccgaggccacccaccccggctcctaccatcgcctcccagcctctgtccctgcgtccggaggcatgtagacccgcagctggtggg

gccgtgcatacccggggtcttgacttcgcctgcgatatctacatttgggcccctctggctggtacttgcggggtcctgctgctttcactcgtga

tcactctttactgtaggagtaagaggagcaggctcctgcacagtgactacatgaacatgactccccgccgccccgggcccacccgcaag

cattaccagccctatgccccaccacgcgacttcgcagcctatcgctcccgcgtgaaattcagccgcagcgcagatgctccagcctacaag

caggggcagaaccagctctacaacgaactcaatcttggtcggagagaggagtacgacgtgctggacaagcggagaggacgggaccc

agaaatgggcgggaagccgcgcagaaagaatccccaagagggcctgtacaacgagctccaaaaggataagatggcagaagcctata

gcgagattggtatgaaaggggaacgcagaagaggcaaaggccacgacggactgtaccagggactcagcaccgccaccaaggacacc

tatgacgctcttcacatgcaggccctgccgcctcgg.

The amino acid sequence of the fusion protein shown in SEQ ID NO. 6 was as follows:

QVQLVESGGGVVQPGRSLRLSCAASGFTFSSYGMHWVRQAPGKGLEWVAVI WYDGSNKFYADSVKGRFTISRDNSKNTLYLQMNSLRAEDTAVYYCARRSDYRGYYGM DVWGQGTTVTVSSGSTSGGGSGGGSGGGGSSEIVLTQSPATLSLSPGERATLSCRASQSV INYLAWYQQKPGQAPRLLIYDASNRASGIPARFSGSGSGTDFTLTISSLEPEDFAVYYCQQ RRNWPLTFGGGTKVEIKGGGGSGGGGSGGGGSQVQLVQSGAEVKKPGASVKVSCKAS GYTFTDFEMHWVRQAPGQGLEWMGDIDPGTGDTAYNLKFKGRVTMTTDTSTSTAYME LRSLRSDDTAVYYCALGAFVYWGQGTLVTVSSGSTSGGGSGGGSGGGGSSDVVMTQS PLSLPVTPGEPASISCRSSQSLANSYGNTYLSWYLQKPGQSPQLLIYGISNRFSGVPDRFS GSGSGTDFTLKISRVEAEDVGVYYCLQGTHQPYTFGQGTKLEIKTTTPAPRPPTPAPTIAS QPLSLRPEACRPAAGGAVHTRGLDFACDIYIWAPLAGTCGVLLLSLVITLYCRSKRSRLL HSDYMNMTPRRPGPTRKHYQPYAPPRDFAAYRSRVKFSRSADAPAYKQGQNQLYNELN LGRREEYDVLDKRRGRDPEMGGKPRRKNPQEGLYNELQKDKMAEAYSEIGMKGERRR GKGHDGLYQGLSTATKDTYDALHMQALPPR.

(2) The two kinds of nucleotide sequences synthesized in step (1) were respectively inserted into pLVX-IRES-ΔNGFR (purchased from Clontech Company, article number 631982) as a vector to obtain two kinds of lentivirus expression vectors of this example.

Example 2

This example was used to explain the preparation and test of T cells expressing a chimeric antigen receptor.

(1) The two kinds of lentivirus expression vectors constructed in Example 1 and an empty pLVX-IRES-ΔNGFR vector were packaged with lentiviruses respectively, and then T cells were cultured in vitro, transfected and proliferated according to the following methods.
(2) T cells in blood were separated according to the following method: 1 mL of sterile PBS and 1 mL of blood were mixed evenly, then slowly added to an upper layer of a lymphocyte separation medium Ficoll, and centrifuged at 4°C and 400 g for 30 min, with acceleration and deceleration set to 0 respectively. After centrifugation, the upper plasma was removed, the middle white membrane cells were pipetted, PBS was added for re-suspension washing, and centrifugation was carried out for 10 min at 100 g, with normal acceleration and deceleration. After centrifugation, the upper washing solution was removed, 1 mL of 1640 + 10% FBS + 1% double antibody + 1 × Glutamine medium was added to re-suspend cells, and then the cells were stimulated to proliferate by means of anti-human CD3/CD28 magnetic beads (purchased from Thermo Fisher company), where the concentration of re-suspended cells was $1 \times 10^6$ cells/mL and the amount of magnetic beads added was 100 μL. Then 100 IU/mL rhIL-2 (Peprotech) was added to stimulate culture for 2 days to obtain T cells.
(3) Transfection of lentiviruses was carried out according to the following method: the lentiviruses packaged in step (1) were respectively added to three parts of T cells separated above, and then polybrene with a final concentration of 6 μg/mL was added, followed by uniform mixing and centrifugation at 32°C and 800 g for 100 min. After centrifugation, the culture was continued in an incubator for 24 h. After the culture, the culture solution was centrifuged at 1500 rpm for 15 min, and the centrifuged cells were inoculated at a density of $1 \times 10^6$/mL into a culture plate and stimulated for culture with rhIL2-100 IU/mL. After that, the medium was changed every 2-3 days until 2-4 weeks, and transfected T lymphocytes CAR-T 1, CAR-T 2 and CAR-T 3 were obtained. CAR-T 1 was transfected with the nucleotide sequence shown in SEQ ID NO. 4 and could express the fusion protein shown in SEQ ID NO. 3; CAR-T 2 was transfected with the nucleotide sequence shown in SEQ ID NO. 5 and could express the fusion protein shown in SEQ ID NO. 6; and CAR-T 3 was transfected with the empty vector.

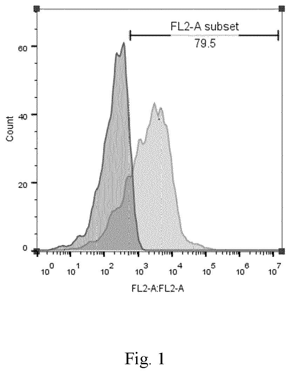

After culture, the cells were re-suspended with PBS, and the ratio of the above three kinds of CAR-T cells and the expression of CAR protein on the surface were tested with a flow cytometer. The test method was as follows: the T cells to be tested after transfection were centrifuged and collected respectively, the supernatant was discarded after the T cells to be tested were washed with PBS once, and a corresponding test amount of monoclonal antibody was added according to antibody instructions, followed by PBS washing, re-suspension, filtration with a membrane, and sandwich test with a flow cytometer, where the antibody used for the test was a mixture of His-tag labeled CD44 and PE labeled anti-His-tag antibody. The results were shown in Figs. 1-3.

Fig. 1 is a flow cytometer test result diagram of CAR-T 1 cells according to an embodiment of the disclosure;

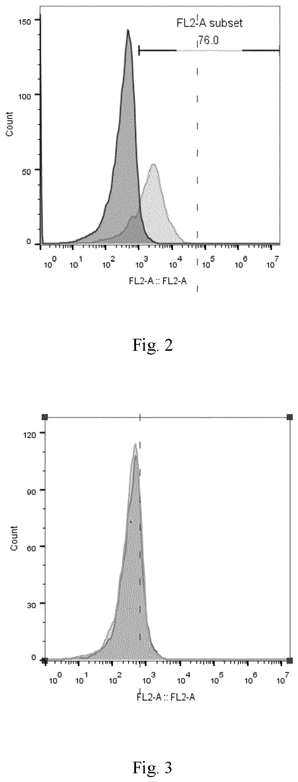

Fig. 2 is a flow cytometry test result diagram of CAR-T 2 cells provided by an embodiment of the disclosure; and

Fig. 3 is a flow cytometry test result diagram of CAR-T 3 cells according to the embodiment of the disclosure.

As can be seen from Fig. 1 and Fig. 2, other cells different from normal T lymphocytes were detected out from the CAR-T 1 cells and the CAR-T 2 cells. As can be seen from Fig. 3, other cells different from normal T lymphocytes were not detected out from the CAR-T 3 cells. This showed that the CAR-T cells transfected with fusion genes in this example had successfully expressed the target fusion protein.

Example 3

This example was used to verify the ability of the CAR-T cells constructed in Example 2 to kill tumor cells.

Three kinds of CAR-T cells obtained by transfection and culture in Example 2 were respectively mixed with CD44 and CD133 positive glioma stem cells GSC20 according to different effect-target ratios (the number of T cells: the number of glioma stem cells). The mixed cells were cultured in 96-well plates, where each well contained $4 \times 10^4$ glioma stem cells, and the reaction system was 200 $\mu$L per well. The culture conditions included: 37°C, 5% $CO_2$, and culture in a saturated humidity incubator for 4 h.

Determination of lactate dehydrogenase activity: after centrifugation, 100 $\mu$L of supernatant was pipetted from each well and placed in 96-well enzyme-labeled plates, 100 $\mu$L of LDH substrate was added to each well, and the reaction was carried out at room temperature for 30 min without light. After the reaction, 50 $\mu$L of termination solution was added to each well to terminate the enzymatic reaction. The optical density (OD) was measured with a Microplate Reader 490 nm. The average optical density (OD) of each group was calculated, and the lysis rate of glioma stem cells by each kind of T cells was calculated according to the following formula. The results were shown in Table 1.

$$\text{Lysis rate\%} = (\text{OD of the experimental group - OD spontaneously released by glioma stem cells - OD naturally released by effector cells})/ (\text{maximum OD released by glioma stem cells - OD spontaneously released by glioma stem cells})$$

Table 1

| CAR-T cells | Lysis rate of glioma stem cells by CAR-T cells under different effect-target ratios (%) | | | |
|---|---|---|---|---|
| | 5:1 | 3:1 | 2:1 | 1:1 |
| CAR-T 1 | 88.57% | 69.12% | 44.71% | 31.75% |
| CAR-T 2 | 80.87% | 48.51% | 40.93% | 30.32% |
| CAR-T 3 | 6.87% | 2.63% | 1.23% | 2.41% |

As can be seen from Table 1, the anti-TIM3 single-chain antibody provided by the disclosure can effectively improve the tumor immunocompetence of T lymphocytes and enhance the ability of T lymphocytes to kill tumor cells.

Comparative Example

Conventional second generation CAR-T cells (EGFR vIII-CD28-CD3) targeting a classical tumor target EGFR vIII were used to replace the T cells in Example 3, and then killing rates of glioma stem cells GSC20 by CAR-T under different effect-target ratios were tested according to the method of Example 3. The test result is shown in Table 2.

Table 2

| | Lysis rate of glioma stem cells by conventional second generation CAR-T cells under different effect-target ratios (%) | | | |
|---|---|---|---|---|
| Effect-target ratio | 5:1 | 3:1 | 2:1 | 1:1 |
| Conventional CAR-T | 17.57% | 14.62% | 13.97% | 11.88% |

As can be seen from Table 2, the ability of the conventional second generation CAR-T cells targeting a classical tumor stem cell target EGFR vIII to kill glioma stem cells was weaker than that of the T cells expressing the anti-TIM3 single-chain antibody provided by the disclosure.

The preferred embodiments of the disclosure are described in detail above with reference to the accompanying drawings. However, the disclosure is not limited to the specific details in the above embodiments. Various simple variations may be made to the technical solutions of the disclosure within the scope of the technical idea of the disclosure, and these simple variations fall within the scope of the disclosure.

It should be further noted that the specific technical features described in the above specific embodiments may be combined in any suitable manner without contradiction. In order to avoid unnecessary repetition, the disclosure will not describe various possible combinations.

Moreover, the various different embodiments of the disclosure may be combined randomly without deviating from the idea of the disclosure, and the combinations should also be regarded as the disclosure of the disclosure.

**Claims**

1. An anti-TIM3 single-chain antibody, wherein the amino acid sequence of the anti-TIM3 single-chain antibody comprises a sequence shown in SEQ ID NO. 1.

2. A nucleic acid, wherein the nucleic acid encodes the anti-TIM3 single-chain antibody according to claim 1; and preferably, the nucleic acid has a nucleotide sequence shown in SEQ ID NO. 2.

3. A fusion protein, wherein the fusion protein contains an antigen binding domain, a transmembrane domain and an intracellular signaling domain that are sequentially linked; the amino acid sequence of the intracellular signaling domain comprises the amino acid sequence of the anti-TIM3 single-chain antibody according to claim 1;

    preferably, the antigen binding domain comprises an anti-CD44 single-chain antibody and/or an anti-CD133 single-chain antibody, and the intracellular signaling domain comprises the anti-TIM3 single-chain antibody; and more preferably, the fusion protein has an amino acid sequence shown in SEQ ID NO. 3.

4. A fusion nucleic acid, wherein the fusion nucleic acid encodes the fusion protein according to claim 3; and preferably, the fusion nucleic acid has a nucleotide sequence shown in SEQ ID NO. 4.

5. An expression vector, wherein the expression vector is inserted with an expression cassette, the expression cassette comprises a first nucleic acid fragment encoding an antigen binding molecule and a second nucleic acid fragment encoding an intracellular signaling molecule, the intracellular signaling molecule contains the anti-TIM3 single-chain antibody according to claim 1, and an IRES element or a 2A peptide encoding sequence is inserted between the first nucleic acid fragment and the second nucleic acid fragment; and preferably, the expression cassette is the fusion nucleic acid according to claim 4.

6. A cell expressing a chimeric antigen receptor, wherein the cell expressing a chimeric antigen receptor is obtained by transfection of the expression vector according to claim 5 by a host cell, and the chimeric antigen receptor contains the anti-TIM3 single-chain antibody according to claim 1.

7. The cell according to claim 6, wherein the host cell is a T cell.

8. Use of the anti-TIM3 single-chain antibody according to claim 1, the nucleic acid according to claim 2, the fusion protein according to claim 3, the fusion nucleic acid according to claim 4, the expression vector according to claim 5, and the cell expressing a chimeric antigen receptor according to claim 6 or 7 in preparing a medicine for treating a tumor.

9. The use according to claim 8, wherein the tumor is a glioma.

10. A pharmaceutical composition, wherein an active ingredient of the pharmaceutical composition comprises the cell expressing an anti-TIM3 chimeric antigen receptor according to claim 6 or 7.

Fig. 1

Fig. 2

Fig. 3

## INTERNATIONAL SEARCH REPORT

| International application No. |
|---|
| **PCT/CN2021/109849** |

### A. CLASSIFICATION OF SUBJECT MATTER

C07K 16/28(2006.01)i; C12N 15/13(2006.01)i; C07K 19/00(2006.01)i; C12N 15/62(2006.01)i; C12N 15/867(2006.01)i; C12N 5/10(2006.01)i; A61K 39/00(2006.01)i; A61P 35/00(2006.01)i

According to International Patent Classification (IPC) or to both national classification and IPC

### B. FIELDS SEARCHED

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS; CNKI; CNTXT; DWPI; SIPOABS; EPTXT; USTXT; WOTXT; JPTXT; ISI Web of Science; CA and search terms: TIM3, TIM-3, HAVCR2, CD366, T细胞免疫球蛋白粘蛋白分子, 抗体, CAR, 嵌合抗原受体, 双特异性, 串联, T-cell, immunoglobulin, mucin, antibod+, chimeric antigen receptor, bispecific, tandem, tanCDR, tCDR et al.; 中国专利生物序列检索系统; Genbank; EMBL; STN and sequence searched: SEQ ID NOs: 1 and 3 et al.

### C. DOCUMENTS CONSIDERED TO BE RELEVANT

| Category* | Citation of document, with indication, where appropriate, of the relevant passages | Relevant to claim No. |
|---|---|---|
| X | CN 109451741 A (SYMPHOGEN AS) 08 March 2019 (2019-03-08) <br> see abstract, claims 1-43, SEQ ID NO: 3 and 4 | 1-2, 8-9 |
| Y | CN 109451741 A (SYMPHOGEN AS) 08 March 2019 (2019-03-08) <br> see abstract, claims 1-43, SEQ ID NO: 3 and 4 | 3-10 |
| Y | WO 2019232444 A1 (UNIVERSITY OF WASHINGTON) 05 December 2019 (2019-12-05) <br> see abstract, claims 1-104 | 3-10 |
| X | CN 110621698 A (SYMPHOGEN AS) 27 December 2019 (2019-12-27) <br> see abstract, claim 14, SEQ ID NO: 3 and 4 | 1-2, 8-9 |
| Y | CN 110621698 A (SYMPHOGEN AS) 27 December 2019 (2019-12-27) <br> see abstract, claim 14, SEQ ID NO: 3 and 4 | 3-10 |
| A | WO 2018156434 A1 (H LEE MOFFITT CANCER CT & RES) 30 August 2018 (2018-08-30) <br> see entire document | 1-10 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 October 2021** | **04 November 2021** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088** <br> **China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2021/109849**

### C.    DOCUMENTS CONSIDERED TO BE RELEVANT

| Category* | Citation of document, with indication, where appropriate, of the relevant passages | Relevant to claim No. |
|---|---|---|
| A | CN 109735500 A (SUZHOU MAOXING BIOTECHNOLOGY CO., LTD.) 10 May 2019 (2019-05-10)<br>    see entire document | 1-10 |
| A | CN 110938146 A (HUAZHONG AGRICULTURAL UNIVERSITY) 31 March 2020 (2020-03-31)<br>    see entire document | 1-10 |

## INTERNATIONAL SEARCH REPORT

| International application No. |
| --- |
| **PCT/CN2021/109849** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

       ☑  in the form of an Annex C/ST.25 text file.

       ☐  on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

       ☐  in the form of an Annex C/ST.25 text file (Rule 13*ter*.1(a)).

       ☐  on paper or in the form of an image file (Rule 13*ter*.1(b) and Administrative Instructions, Section 713).

2.  ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**
Information on patent family members

| International application No. |
| --- |
| **PCT/CN2021/109849** |

| Patent document cited in search report | | | Publication date (day/month/year) | Patent family member(s) | | | Publication date (day/month/year) |
| --- | --- | --- | --- | --- | --- | --- | --- |
| CN | 109451741 | A | 08 March 2019 | AU | 2017251250 | A1 | 18 October 2018 |
| | | | | CO | 2018010458 | A2 | 10 October 2018 |
| | | | | EA | 201892294 | A1 | 30 April 2019 |
| | | | | MA | 44659 | A | 14 April 2021 |
| | | | | TN | 2018000333 | A1 | 16 January 2020 |
| | | | | CA | 3020647 | A1 | 19 October 2017 |
| | | | | SG | 10201912937 S | A | 27 February 2020 |
| | | | | KR | 20180133482 | A | 14 December 2018 |
| | | | | EP | 3443009 | A1 | 20 February 2019 |
| | | | | EP | 3443009 | B1 | 08 September 2021 |
| | | | | US | 2019276531 | A1 | 12 September 2019 |
| | | | | BR | 112018070919 | A2 | 29 January 2019 |
| | | | | TW | 201736397 | A | 16 October 2017 |
| | | | | PE | 20181805 | A1 | 19 November 2018 |
| | | | | JP | 2019519199 | A | 11 July 2019 |
| | | | | SG | 11201808724 S | A | 29 November 2018 |
| | | | | MX | 2018012076 | A | 20 February 2019 |
| | | | | PH | 12018502112 | A1 | 23 September 2019 |
| | | | | WO | 2017178493 | A1 | 19 October 2017 |
| | | | | CL | 2018002878 | A1 | 26 April 2019 |
| | | | | IL | 262176 | D0 | 29 November 2018 |
| WO | 2019232444 | A1 | 05 December 2019 | KR | 20210016431 | A | 15 February 2021 |
| | | | | SG | 11202011383V | A | 30 December 2020 |
| | | | | US | 2020071397 | A1 | 05 March 2020 |
| | | | | CA | 3101505 | A1 | 05 December 2019 |
| | | | | IL | 279063 | D0 | 31 January 2021 |
| | | | | EP | 3802798 | A1 | 14 April 2021 |
| | | | | CN | 112912493 | A | 04 June 2021 |
| | | | | JP | 2021525524 | A | 27 September 2021 |
| | | | | AU | 2019279021 | A1 | 10 December 2020 |
| CN | 110621698 | A | 27 December 2019 | CA | 3058960 | A1 | 11 October 2018 |
| | | | | TW | 201841652 | A | 01 December 2018 |
| | | | | CL | 2019002850 | A1 | 13 March 2020 |
| | | | | WO | 2018185232 | A1 | 11 October 2018 |
| | | | | SG | 11201908919 X | A | 30 October 2019 |
| | | | | MA | 49042 | A | 12 February 2020 |
| | | | | US | 2020407444 | A1 | 31 December 2020 |
| | | | | IL | 269644 | D0 | 28 November 2019 |
| | | | | JP | 2020513009 | A | 30 April 2020 |
| | | | | PH | 12019502191 | A1 | 15 June 2020 |
| | | | | KR | 20190137105 | A | 10 December 2019 |
| | | | | AU | 2018247916 | A1 | 17 October 2019 |
| | | | | EP | 3606957 | A1 | 12 February 2020 |
| | | | | SG | 10201912941 P | A | 27 February 2020 |
| | | | | PE | 20191741 | A1 | 12 December 2019 |
| | | | | EA | 201992350 | A1 | 23 March 2020 |
| | | | | MX | 2019011961 | A | 05 December 2019 |
| | | | | CO | 2019012080 | A2 | 17 January 2020 |
| | | | | BR | 112019020662 | A2 | 05 May 2020 |
| | | | | RU | 2019134934 | A | 05 May 2021 |

Form PCT/ISA/210 (patent family annex) (January 2015)

INTERNATIONAL SEARCH REPORT
Information on patent family members

International application No.

**PCT/CN2021/109849**

| Patent document cited in search report | | | Publication date (day/month/year) | Patent family member(s) | | | Publication date (day/month/year) |
|---|---|---|---|---|---|---|---|
| WO | 2018156434 | A1 | 30 August 2018 | EP | 3585403 | A1 | 01 January 2020 |
| | | | | EP | 3585403 | A4 | 09 December 2020 |
| | | | | US | 2019374579 | A1 | 12 December 2019 |
| CN | 109735500 | A | 10 May 2019 | None | | | |
| CN | 110938146 | A | 31 March 2020 | None | | | |

Form PCT/ISA/210 (patent family annex) (January 2015)